# EUROPÄISCHE PATENTSCHRIFT

(11) **EP 1 149 110 B1**
(45) Veröffentlichungstag und Bekanntmachung des Hinweises auf die Patenterteilung: **04.06.2003**
(21) Anmeldenummer: 99964446.1
(22) Anmeldetag: 17.12.1999
(51) Int. Cl.: C07H 21/00, C07H 19/06, C07H 19/16

(54) **VERFAHREN ZUR LICHTGESTEUERTEN BIOCHIP-SYNTHESE**
METHOD FOR THE LIGHT-CONTROLLED SYNTHESIS OF BIOCHIPS
PROCEDE DE SYNTHESE COMMANDEE PAR LA LUMIERE DE BIOPUCES

(30) Priorität: 17.12.1998 DE 19858440
(43) Veröffentlichungstag der Anmeldung: 31.10.2001
(73) Patentinhaber: Deutsches Krebsforschungszentrum Stiftung des öffentlichen Rechts, 69120 Heidelberg (DE)
(72) Erfinder: BEIER, Markus, D-69120 Heidelberg (DE); MATYSIAK, Stefan, D-69118 Heidelberg (DE); HOHEISEL, Jörg, D-69168 Wiesloch (DE)
(74) Vertreter: Schüssler, Andrea, Dr.
(86) Internationale Anmeldenummer: DE9904051
(87) Internationale Veröffentlichungsnummer: WO00035931

(56) Entgegenhaltungen:
- US-A- 5 763 599
- M. PFISTER ET AL.: "The 2-(4-nitrophenyl)ethylsulfonyl (Npes) group: a new type of protection in nucleoside chemistry" HELVETICA CHIMICA ACTA, Bd. 78, 1995, Seiten 1705-1737, XP002148304

## Beschreibung

Die vorliegende Erfindung betrifft ein Verfahren zum lichtgesteuerten (photolithografischen) Aufbau von Biochips.

Das Gebiet der Synthese von Oligonukleotiden und DNA- bzw. RNA-Chips hat seit einigen Jahren einen ungeheuren Aufschwung genommen und es werden in diesem Zusammenhang effiziente Methoden benötigt.

Heute werden synthetisch DNA-Fragmente (Oligonucleotide) fast ausschließlich nach der Phosphitamid-Methode dargestellt. Dies erfolgt unter Zuhilfenahme von sog. DNA-Synthesizern. Diese Maschinen synthetisieren nach Eingabe der Zielsequenz automatisch das gewünschte DNA-Oligomer. Hierzu verfügt der DNA-Synthesizer über eine computergesteuerte Programmierung, die festlegt in welcher Abfolge bestimmte Reagenzien auf die Synthesesäule gepumpt werden, wo dann die eigentliche Synthese des DNA-Oligomers an der festen Phase auf Glaskügelchen aus z.B. controlled pore glass (CPG) stattfindet. Unter den Reagenzien sind u.a. die 4 (geschützten) Phosphitamide für die 4 Basen (Adenin, Cytidin, Guanosin, Thymin), der Katalysator (üblicherweise Tetrazol), ein Oxidationsmittel (üblicherweise Jod), Capping-Reagenz A & B (üblicherweise Essigsäureanhydrid, N-Methylimidazol) und Detritylierungsreagenz (üblicherweise Trichloressigsäure) zu verstehen. Ein Reaktionszyklus besteht nun in der Abfolge von:

| | | |
|---|---|---|
| (1) | Detritylierung | Abspaltung der temporären 5'-O-Dimethoxytrityl-Schutzgruppe durch Trichloressigsäure ; |
| (2) | Kondensation | auf die freigesetzte 5'-Hydroxyl-Funktion wird das der Zielsequenz entsprechende Phosphitamid aufkondensiert; dies geschieht unter Zuhilfenahme des sauren Katalysators (Tetrazol); |
| (3) | Oxidation | die unstabile Phosphittriester-Verknüpfung (P-III) wird durch Oxidation mit Jod in einen stabilen Phosphortriester (P-V) überführt; |
| (4) | Capping | durch einen Acetylierungsschritt (Essigsäureanhydrid + N-Methylimidazol) sollen die 5'-Hydroxyl-Funktionen abgefangen werden, die im vorangegangenen Schritt nicht reagiert haben; das soll ein unkontrolliertes Wachsen des DNA-Strangs verhindern. |

Nachfolgend werden die Schritte (1) bis (4) solange wiederholt, bis die Zielsequenz erreicht ist.

Das Startnucleosid, das vorzusgweise eine 5'-O-Dimethoxytrityl-Schutzgruppe trägt, befindet sich üblicherweise bereits auf dem Träger, so daß mit dem Detritylierungsschritt begonnen wird. Ist die Zielsequenz erreicht, müssen noch die Phosphatschutzgruppen und die Schutzgruppen der exocyclischen Amino-Funktionen der Heterobasen abgespalten werden. Dies erfolgt üblicherweise mittels Ammoniak. Gleichzeitig erfolgt mit Ammoniak auch die Abspaltung des Oligomers vom Träger, so daß das fertigsynthetisierte Oligomer sich dann in der ammoniakalischen Abspaltlösung befindet. Nach Evaporation der ammoniakalischen Lösung wird das Ziel-Oligomer erhalten.

Der für die Qualität der Synthese entscheidende Schritt ist die Kondensation, da alle anderen Schritte (Detritylierung, Oxidation, Capping) quantitativ verlaufen. Die Kondensation wird unter strengstem Ausschluß von Feuchtigkeit durchgeführt. Man kann üblicherweise Kondensationsausbeuten von bis zu 99% erreichen.

Nach einer analogen Methode erfolgt der Aufbau von Nucleinsäure-Chips auf Trägeroberflächen, so daß sich prinzipiell die nachfolgend beschriebenen Chip-Aufbau Methoden auch zur Darstellung von Oligonukleotiden eignen, wenn diese von der Trägeroberfläche abgelöst werden.

Vor einiger Zeit hat sich für die Nukleinsäure-Chip-Synthese eine weitere Methode entwickelt: die lichtgesteuerte Chip-Synthese. Die bei der lichtgesteuerten Synthese verwendeten Phosphitamide (A, C, G, T) haben die gleiche chemische Struktur wie bei der "normalen" DNA-Synthese am handelsüblichen DNA-Synthesizer, mit dem einzigen Unterschied, daß die 5'-säurelabile Dimethoxytritylschutzgruppe durch eine photolabile Schutzgruppe ersetzt ist, die sich sowohl an der 5'- oder 3'-Position befinden kann. Es ergibt sich somit folgender Reaktionsablauf am DNA-Chip-Synthesizer:

| | | |
|---|---|---|
| (1) | Bestrahlung | Abspaltung der temporären photolabilen-Schutzgruppe durch Bestrahlung mit Licht der entsprechenden Wellenlänge; |
| (2) | Kondensation | auf die freigesetzte Hydroxyl-Funktion wird das der Zielsequenz entsprechende Phosphitamid aufkondensiert; dies geschieht unter Zuhilfenahme des sauren Katalysators (Tetrazol); |
| (3) | Oxidation | die unstabile Phosphittriester-Verknüpfung (P-III) wird durch Oxidation mit Jod in einen stabilen Phosphortriester (P-V) überführt; |
| (4) | Capping | durch einen Acetylierungsschritt (Essigsäureanhydrid + N-Methylimidazol) sollen die Hydroxyl-Funktionen abgefangen werden, die im vorangegangenen Schritt nicht reagiert haben; das soll ein unkontrolliertes Wachsen des DNA-Strangs verhindern. |

Nachfolgend werden die Schritte (1) bis (4) solange wiederholt, bis die Zielsequenz erreicht ist.

Das Startnucleosid befindet sich üblicherweise nicht auf dem Träger, so daß hier mit dem Kondensationsschritt begonnen wird. Ist die Zielsequenz erreicht, müssen noch die Phosphatschutzgruppen und die Schutzgruppen der exocyclischen Amino-Funktionen abgespalten werden. Dies erfolgt mittels Ammoniak, aber unter milderen Bedingungen (2 h), damit die auf dem Chip synthetisierten DNA-Stränge nicht vom Träger abgespalten werden. Dies wird dadurch möglich, daß als Schutzgruppen der exocyclischen Amino-Funktionen labilere Schutzgruppen als bei der üblichen Synthese an CPG-Materialien verwendet werden. Der DNA-Chip wird im folgenden einfach der ammoniakalischen Lösung entnommen, mit Wasser gewaschen und kann sofort für Hybridisierungs-Experimente verwendet werden.

Die Qualität der DNA-Chip-Synthese wird bei der lichtgesteuerten Methode nicht nur allein durch die Kondensation, sondern v.a. durch die Effizienz der Abspaltung der photolabilen Schutzgruppen bestimmt, die selten so wirksam ist wie die Abspaltung der Dimethoxytritylschutzgruppe durch Säure. Da im Kondensationsschritt üblicherweise Ausbeuten von bis zu 95-99% erreicht werden können, wird die Qualität des DNA-Chips quasi nur noch von der Effizienz der Photoschutzgruppen-Abspaltung bestimmt.

In der WO 96/18634 und WO 97/44345 sind spezielle photolabile Schutzgruppen vom 2-(2-nitrophenyl-)ethyl-Typ gezeigt, die sich zur Darstellung von Oligonukleotiden auf einem DNA-Chip eignen sollen. Von den Erfindern wurde aber herausgefunden, daß die in diesen Anmeldungen gezeigten photolabilen Schutzgruppen nur sehr schwer mit den üblichen Methoden abzuspalten sind und die Darstellung von DNA-oder RNA-Chips nicht sehr effizient ist. Außerdem stellte sich heraus, daß sich derart dargestellte Chips aufgrund der minderen Qualität nicht mit Fluoreszenz detektieren lassen, was der gegenwärtige Standard ist, um DNA-Chips zu detektieren. Für die Detektion dieser Chips würde eine Sondenmarkierung mittels Radiographie benötigt, was für eine kommerzielle Verwertung unbrauchbar ist.

Die Aufgabe der vorliegenden Erfindung besteht nun darin, ein Verfahren bereitzustellen, mit dem hochwertige Biochips, insbesondere DNA- oder RNA-Chips, herstellbar sind.

Diese Aufgabe wird durch die Gegenstände der Patentansprüche gelöst.

Insbesondere wird diese Aufgabe durch ein Verfahren zur lichtgesteuerten Biochip-Synthese gelöst, bei der photolabile Schutzgruppen vom 2-(2-nitrophenyl-)ethyl-Typ eingesetzt werden, wobei der bei der lichtgesteuerten Chip-Synthese übliche Bestrahlungsschritt in Anwesenheit einer Base durchgeführt wird.

Das erfindungsgemäße Verfahren zur lichtgesteuerten Biochip-Synthese bietet den Vorteil, daß die effiziente Abspaltung von photolabilen Schutzgruppen vom 2-(2-nitrophenyl)-ethyl-Typ erreicht werden kann. Die photolabilen Schutzgruppen haben bevorzugt folgende Formel: 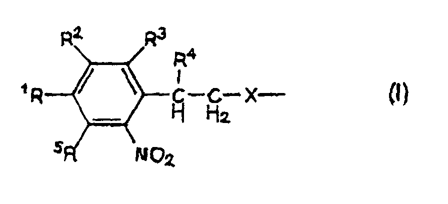
- R¹=: H, NO₂, CN, OCH₃, Halogen, Alkyl oder Alkoxyalkyl mit 1 bis 4 C-Atomen
- R²⁼: H, OCH₃, Alkylrest mit 1 bis 4 C-Atomen oder ggf. substituierter Arylrest
- R³=: H, F, C1, Br, NO₂, Alkylrest mit 1 bis 4 C-Atomen oder ggf. ein Arylrest oder ein aliphatischer Acylrest mit 2 bis 5 C-Atomen
- R⁴=: H, Halogen, OCH₃, Alkylrest mit 1 bis 4 C-Atomen oder ggf. substituierter Arylrest
- R⁵=: H, NO₂, CN, OCH₃, Halogen, Alkyl oder Alkoxyalkyl mit 1 bis 4 C-Atomen oder ggf. substituierter Arylrest
- X=: SO₂ (Sulfonyl), OCO (Oxycarbonyl)

Ganz besonders bevorzugt werden die Schutzgruppen NPPOC oder CNPOC, die die folgende Formel haben, verwendet, wobei NPPOC am bevorzugtesten ist.

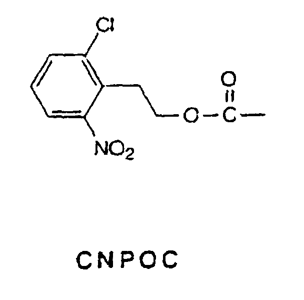

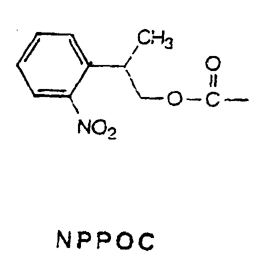

Die obigen Schutzgruppen können allgemein zum Schutz von Hydroxyl- oder Amino-Funktionen an Nucleinsäurederivaten, Peptide, Proteinen, Antikörpern oder anderen Biomolekülen eingesetzt werden, bevorzugt zum Schutz der Hydroxyl-Funktionen in Nucleinsäurederivaten, ganz besonders zum Schutz der 5'-OH Position oder der 3'-OH Position in Nucleinsäurederivaten. Sie sollen, wie bereits im oben genannten Stand der Technik ausgeführt, besonders bei der Synthese von hochwertigen DNA-oder RNA-Chips Verwendung finden, was aber bisher noch nicht gelingt, da der Schritt der Photodeprotektion durch Bestrahlung nicht effizient genug verläuft. Als hochwertige DNA-Chips sollen solche verstanden werden, die erhalten werden, wenn die Ausbeute bei jedem Schritt während der lichtgesteuerten Synthese möglichst hoch ist.

Unter einem Biochip sollen erfindungsgemäß auf einem Träger aufgebaute Biomoleküle, wie DNA, RNA. Nucleinsäureanaloga (z.B. PNA, LNA), Proteine, Peptide, Antikörper, wobei die drei erstgenannten Gruppen bevorzugt sind, verstanden werden.

Erfindungsgemäß ist jegliche(r) auf diesem Gebiet übliche Träger bzw. Matrix bei der Biochip-Herstellung einsetzbar. Dies sind insbesondere Glas, Folien bzw. Membranen aus Polypropylen, Nylon, Cellulose, Cellulosederivate (z.B. Celluloseacetat, Cellulose-Mischester), Polyethersulfonen, Polyamiden, Polyvinylchlorid, Polyvinylidenfluorid, Polyester, Teflon oder Polyethylen. Die Trägeroberflächen können auch mit funktionellen Gruppen versehen sein, z.B. eine Amino-Gruppe, Hydroxyl-Gruppe, Carboxyl-Gruppe, Carbonyl-Gruppe, Thiol-, Amid- oder Phosphat-Gruppe tragen. In einer bevorzugten Ausführungsform weisen die Trägeroberflächen eine Derivatisierung gemäß der deutschen Patentanmeldung 198 53 242.3 auf.

Beim erfindungsgemäßen Verfahren zur lichtgesteuerten Biochip-Synthese werden die Schritte Kondensation, Oxidation und Capping wie üblich (Fodor et al., Science 1991, 251, S. 767 ff.) durchgeführt. Erfindungsgemäß findet allerdings der erste Schritt der Synthese, nämlich die Bestrahlung, unter Zusatz von Basen, bevorzugt starken Basen, insbesondere nichtnukleophilen Basen, statt, was in Zusammenwirken mit dem bei der Bestrahlung angewendeten Licht zu einer überraschend effektiven Abspaltung der Schutzgruppen führt. Die Erfindung sieht vor, durch Beimengung von Basen während der Bestrahlung eine höhere Ausbeute am festen Trägermateriel zu erzielen. Hierbei muß betont werden, daß eine effiziente Abspaltung der Schutzgruppen am festen Trägermaterial weder durch Lichteinwirkung ohne Basenzusatz oder Basenzusatz allein erreicht werden kann. Entsprechend nutzt das erfindungsgemäße Verfahren ein wohl definiertes System aus Lichteinwirkung in Kombination mit Basenzugabe für die erfolgreiche lichtgesteuerte Parallelsynthese von DNA-Arrays am festen Trägermaterial.

Eine solche beispielhafte erfolgreiche Kombination von Lichteinwirkung und Basenzusatz stellt z.B. die Bestrahlung in Anwesenheit von 0,05 M Diisopropylethylamin in Acetonitril dar. So kann die NPPOC-Photoschutzgruppe weder durch Bestrahlung in diversen Lösungsmitteln ohne Basenzusatz (z.B. in Acetonitril allein) effizient abgespalten werden, noch kann diese durch Behandlung mit 0,05 M Diisopropylethylamin in Acetonitril ohne Lichteinwirkung abstrahiert werden. Dies bedeutet, daß nur die Kombination einer Base zusammen mit Lichteinwirkung eine effiziente Abspaltung der Schutzgruppen bewirkt.

In einer bevorzugten Ausführungsforn eignen sich als Zusatz während der Bestrahlung Basen, wie z.B. DBU (1,8-Diazabicyclo[5.4.0]undec-7-en), Diisopropylethylamin, N-Methylmorpholin, N-Methylimidazol, Piperidin. Desweiteren eignen sich die dem Fachmann bekannten sekundären oder tertiären Basen, z.B. DBN (1,5-Diazabicyclo[4.3.0]non-5-en), Triethylamin, DABCO, 2,6-Lutidin, Collidin, Morpholin, Diisopropylamin. Triethylamin.

Die Bestrahlung kann unter den üblichen Bedingungen stattfinden. Die Wellenlänge der Bestrahlung ist von der verwendeten Schutzgruppe abhängig. Die geeigneten Wellenlängen sind dem Fachmann bekannt. So eignet sich zur Abspaltung der NPPOC- oder CNPOC-Schutzgruppe eine 5 minütige Bestrahlung mit einer 100 W Hg-Lampe bei einer Wellenlänge zwischen 320 und 380 nm, bevorzugt bei 365 nm.

Die Menge an während der Bestrahlung anwesender Base variiert zwischen 0,01 M und 1,0 M und ist natürlich von der Basenstärke abhängig. So hat sich es sich bewährt 0,03 bis 1 M (bevorzugt 0,05 bis 0,5 M) DBU in Acetonitril, 0,03 bis 0,8 M (bevorzugt 0,05 M) Diisoproyplethylamin in Acetronitril oder 0,03 bis 1 M (bevorzugt 0,5 M) Piperidin in Acetonitril zu verwenden.

In weiteren bevorzugten Ausführungsformen finden auch bei den anderen Schritten der photolithografischen Nukleinsäure-Chip-Synthese Modifikationen statt. So hat es sich als bevorzugt herausgestellt bei der Kondensation als Katalysator statt Tetrazol Pyridinhydrochlorid (bevorzugt 0,1 bis 1,0 M, ganz bevorzugt 0,5 M, in Acetonitril) einzusetzen. Außerdem wurde ermittelt, daß beim Oxidationsmittel auf den bisher üblichen Zusatz von THF verzichtet werden kann. Auch eine Verminderung der üblichen Jodmenge beim Oxidationsschritt hat sich als vorteilhaft erwiesen. Außerdem wurde beim Capping-Reagenz das übliche THF durch Acetonitril ersetzt. Desweiteren kann die Capping-Prozedur nachweislich ganz unterbleiben, ohne eine negative Auswirkung auf die Qualität der Chips zu haben. Dadurch kann die Synthese um einiges beschleunigt werden.

Die Erfindung wird weiter anhand der nachfolgenden Figuren beschrieben:
- Fig. 1:: Reaktionsschema für die DNA-Chip-Synthese zum Aufbau eines 8-mers ( C A G G T C G C ) auf einer Glasträgeroberfläche
- Fig. 2:: NPPOC-Photochemie; konventionell (ohne Basenzusatz bzw. zuwenig Base) → keine DNA-Chip-Synthese
- Fig. 3:: NPPOC-Photochemie; Bestrahlung unter Zusatz von Basen → effektive DNA-Chip-Synthese

Die Erfindung wird weiter anhand des nachfolgenden Beispiels beschrieben:

### Beispiel 1: Lichtgesteuerte (photolithografische) DNA-Chip-Synthese unter Verwendung von NPPOC als Schutzgruppe

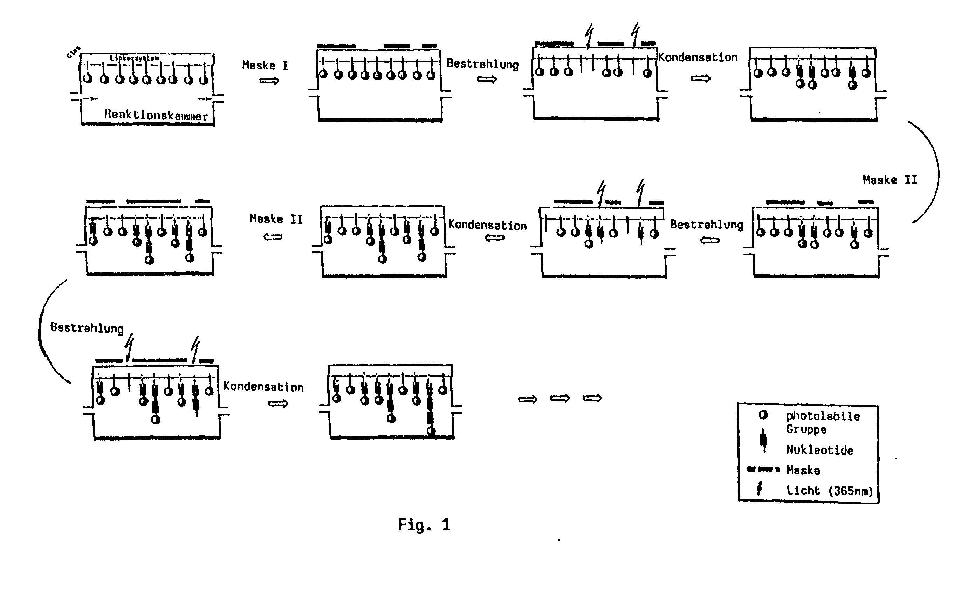

Die DNA-Chip-Synthese wurde analog dem bekannten Verfahren von Fodor et al. (Science 1991, 251. S. 767 ff) auf einer Glasoberfläche als Träger durchgeführt. Der Reaktionsablauf ist schematisch in Fig. 1 gezeigt. Als Nucleoside wurden NPPOC-geschützte Phosphitamide (Fa. Nigu-Chemie, Waldkraiburg) eingesetzt.

Eine Programm am DNA-Chip-Synthesizer setzt sich aus einzelnen Unterprogrmammen (Subroutines) zusammen, die entsprechend die einzelnen Syntheseschritte bei der Oligonucleotidsynthese gemäß der Phosphitamidchemie repräsentieren und nacheinander abgearbeitet werden. Der Zyklus ist wie folgt:
(1) M-wash
(2) M-coup
(3) M-wash
(4) M-ox
(5) M-wash
(6) M-dry
(7) M-Bestrahl
(8) M-waitdr.
(9) M-wash
(10) M-Bestrahl
(11) M-waitdr.
(12) M-wash

| | |
|---|---|
| (1), (3), (5), (9), (12) = M-wash | Unterprogramm, das zwischen alle anderen zwischengeschaltet ist, zum Zweck, die Reaktionsdurchflußkammer zu waschen und Rückstände aus dem vorigen Schritt zu entfernen |
| | |
| (2) = M-coup | Kopplung des neuen Phosphitamidbausteins auf die durch Licht freigesetzte 5'-OH Funktion des vorangegangenen Bausteins in dieser Position |
| | |
| (3) = M-ox | Labile Phosphit-Triesterfunktion wird durch Jod zum Phosphat-Triester aufoxidiert |
| | |
| (6) = M-dry | Löst das Öffnen des Shutters aus und dadurch die Bestrahlung der Reaktionsdurchflußkammer mit Licht. Eine externe elektronische Schaltung, die vom DNA-Synthesizer über ein potentialfreies Signal angesprochen wird, löst das Öffnen aus. An dieser Schaltung wird eingestellt, wie lange der Shutter geöffnet bleibt und somit wie lange bestrahlt wird. Die Bestrahlungsprozedur wird nachfolgend ausführlicher beschrieben. |
| | |
| (7), (10) = M-Bestrahl | Die Bestrahlungslösung wird in die Reaktionsdurchflußkammer gepumpt. In einigen Fällen enthält die Bestrahlungslösung eine Base (DBU, Piperidin, ...) |
| | |
| (8), (11) = M-waitdr. | Warteschleife von 110 sec. (kann beliebig eingestellt werden), beide Subroutinen zusammen ergeben eine gewünschte Bestrahlungsdauer (hier: 5 Minuten) |

Die Bestrahlung (5 Min. Bestrahlung mit 100 W Hg-Lampe, bei 365 nm) wurde wie folgt durchgeführt:
- Öffnen des Shutters (dadurch Beginn der Bestrahlung in der Reaktionskammer)
- Fluten der Reaktions-Durchflußkammer mit Bestrahlungslösung
- nach 150 sec. wird die Bestrahlungslösung aus der Kammer herausgepumpt
- kurzes Waschen der Kammer mit Acetonitril
- erneutes Fluten der Reaktions-Durchflußkammer mit Bestrahlungslösung - nach insgesamt 300 sec. schließt der Shutter (die Belichtung ist beendet)
- die Bestrahlungslösung wird aus der Kammer herausgepumpt
- gründliches Waschen der Kammer mit Acetonitril

Die nachfolgenden Schritte der Kondensation, Oxidation und Capping waren gegenüber dem Reaktionsablauf des Standes der Technik (Weiler et al., Analytical Biochemistry, 243, S. 218-227 (1996)) unverändert oder wiesen die auf Seite 8 beschriebenen Modifikationen auf.

Die Bestrahlungslösungen enthielten 0,05 M DBU, 0,5 M DBU, 0,05 M Diisopropylethylamin oder 0,05 M Piperidin, jeweils in Acetonitril.

Als Kontrolle wurden Chips unter Verwendung von NPPOCgeschützten Phosphitamiden, jedoch ohne Verwendung von Base bzw. zuwenig Base im Bestrahlungschritt, hergestellt. Die Bestrahlungslösungen enthielten in diesen Fällen 0,005 M DBU in Acetonitril, reines Acetonitril oder 10% H₂O/MeOH. In einem Falle wurde die Bestrahlung trocken durchgeführt.

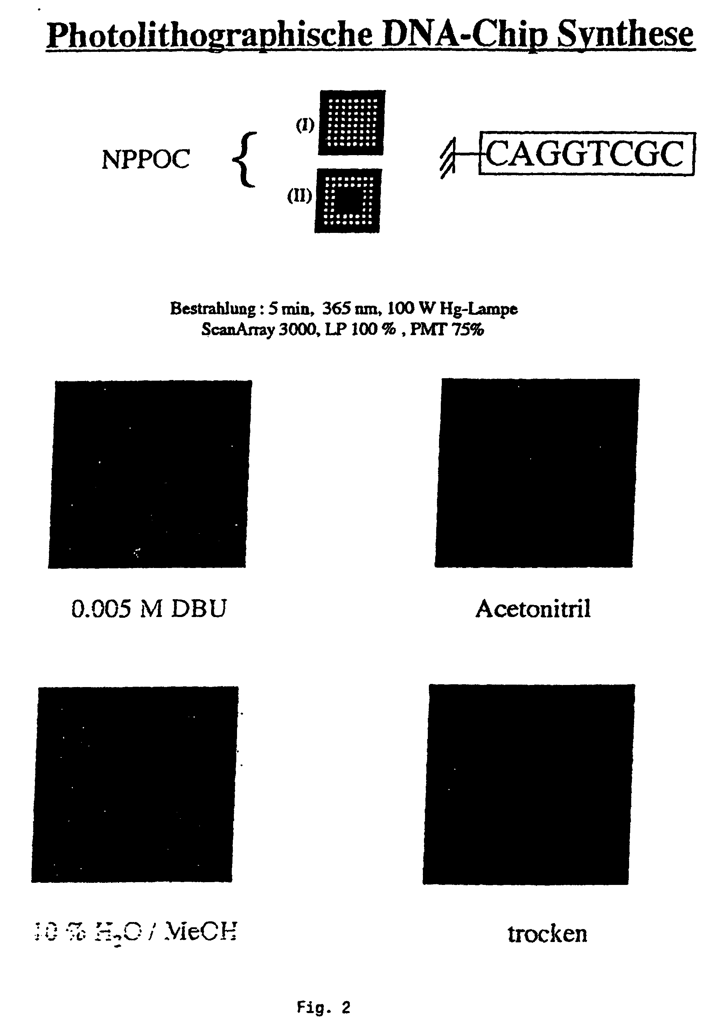

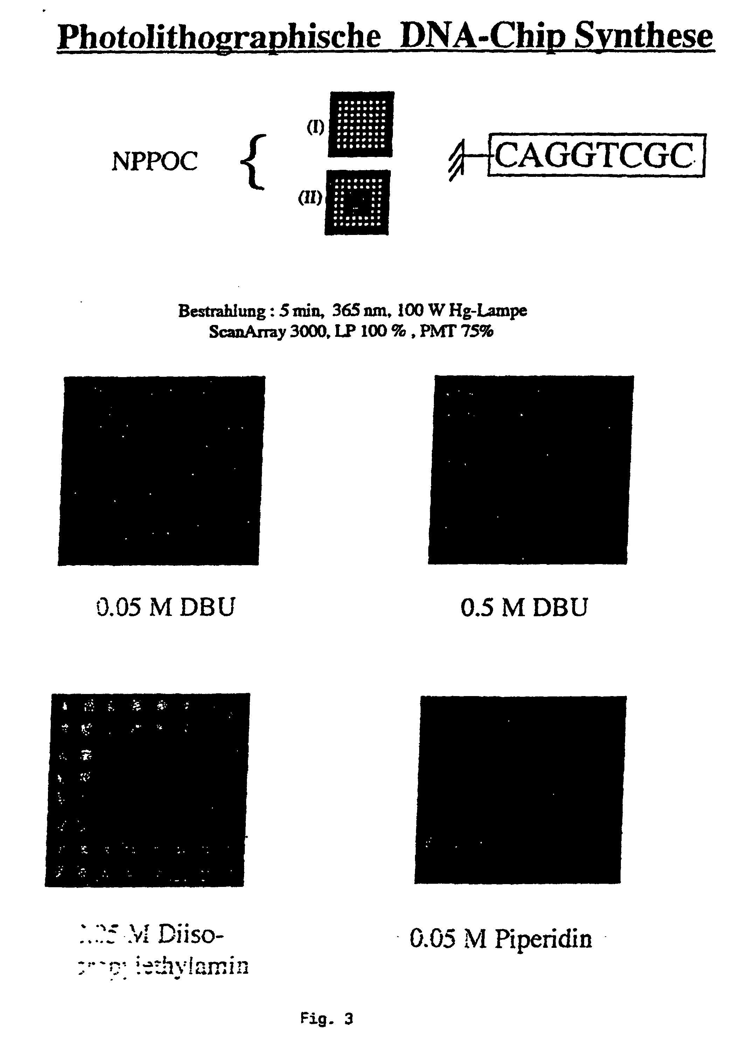

Beim Vergleich der Figuren 2 und 3, die eine Fluoreszenzanalyse der hergestellten Chips darstellen, ist zu sehen, daß nur mit Basenadditiv bei der Bestrahlung eine effektive DNA-Chip-Synthese möglich ist, d.h. nur dort ist ein der Maske II entsprechendes Muster zu erkennen. Ohne oder bei zuwenig Basenzusatz wird keine Chipsynthese erhalten, was durch die total geschwärzten Images ausgedrückt wird. Es ist damit belegt, daß der Zusatz der Base aktiv bei der Abstraktion der Photoschutzgruppe vom Typ der Formel (I) hilft.

## Patentansprüche

1. Verfahren zur lichtgesteuerten Biochip-Synthese bei der photolabile Schutzgruppen vom 2-(2-nitrophenyl-) ethyl-Typ eingesetzt werden, wobei der bei der photolithografischen Chip-Synthese übliche Bestrahlungsschritt in Anwesenheit einer Base durchgeführt wird.

2. Verfahren nach Anspruch 1, wobei die Base eine sekundäre oder tertiäre Base ist.

3. Verfahren nach Anspruch 1 oder 2, wobei die Base ausgewählt ist aus DBU (1,8-Diazabicyclo[5.4.0]undec-7-en, DBN (1,5-Diazabicyclo[4.3.0]non-5-en, Diiisopropylethylamin, Pyridin, Piperidin, Triethylamin, Diisopropylamin, N-Methylmorpholin, 2,6-Lutidin, Collidin, N-Methylimidazol, Dabco, N,N,-Dimethylaminopyridin.

4. Verfahren nach einem der Ansprüche 1 bis 3, wobei die Base in einer Konzentration von 0,01 bis 1 M verwendet wird.

5. Verfahren nach einem der Ansprüche 1 bis 4, wobei die photolabile Schutzgruppe die Formel (I) hat: 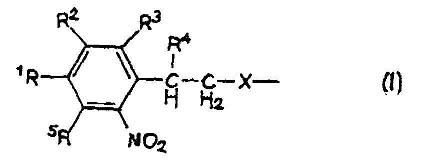
R¹= H, NO₂, CN, OCH₃, Halogen, Alkyl oder Alkoxyalkyl mit 1 bis 4 C-Atomen
R²= H, OCH₃, Alkylrest mit 1 bis 4 C-Atomen oder ggf. substituierter Arylrest
R³= H, F, Cl, Br, NO₂, Alkylrest mit 1 bis 4 C-Atomen oder ggf. ein Arylrest oder ein aliphatischer Acylrest mit 2 bis 5 C-Atomen
R⁴= H, Halogen, OCH₃, Alkylrest mit 1 bis 4 C-Atomen oder ggf. substituierter Arylrest
R⁵= H, NO₂, CN, OCH₃, Halogen, Alkyl oder Alkoxyalkyl mit 1 bis 4 C-Atomen oder ggf. substituierter Arylrest
X= SO₂ (Sulfonyl), OCO (Oxycarbonyl)

6. Verfahren nach einem der vorhergehenden Ansprüche, wobei die photolabile Schutzgruppe NPPOC oder CNPOC ist.

7. Verfahren nach einem der vorhergehenden Ansprüche, wobei der Biochip ein Nukleinsäure- oder Nukleinsäureanaloga-Chip ist.

8. Verfahren nach Anspruch 7, wobei der Nukleinsäure-Chip ein DNA- oder RNA-Chip ist.

9. Verfahren nach Anspruch 7, wobei der Nukleinsäureanaloga-Chip ein PNA- oder LNA-Chip ist.

10. Verfahren nach einem der vorhergehenden Ansprüchen, wobei zusätzlich der bei der lichtgesteuerten Chip-Synthese übliche Kondensationsschritt mit Pyridin-Hydrochlorid als Katalysator durchgeführt wird.

## Claims

1. A method for the light-controlled biochip synthesis in which photolabile protecting groups of the 2-(2-nitrophenyl)ethyl type are used, wherein the irradiation step common for the photolithographic chip synthesis is carried out in the presence of a base.

2. The method according to claim 1, wherein the base is a secondary or tertiary base.

3. The method according to claim 1 or 2, wherein the base is selected from the group consisting of DBU (1,8-diazabicyclo[5.4.0]undec-7-ene, DBN (1,5-diazabicyclo[4.3.0]none-5-ene, diisopropylethylamine, pyridine, piperidine, triethylamine, diisopropylamine, N-methylmorpholine, 2,6-lutidine, collidine, N-methylimidazole, dabco, N,N-dimethylaminopyridine.

4. The method according to any one of claims 1 to 3, wherein the base is used in a concentration of 0.01 to 1 M.

5. The method according to any one of claims 1 to 4, wherein the photolabile protecting group has the formula (I): 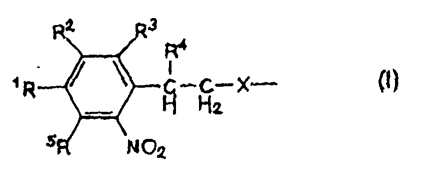
R¹ = H, NO₂, CN, OCH₃, halogen, alkyl or alkoxyalkyl having 1 to 4 C atoms,
R² = H, OCH₃, alkyl residue having 1 to 4 C atoms or optionally substituted aryl residue,
R³ = H, F, Cl, Br, NO₂, alkyl residue having 1 to 4 C atoms or optionally an aryl residue or an aliphatic acyl residue having 2 to 5 C atoms,
R⁴ = H, halogen, OCH₃, alkyl residue having 1 to 4 C atoms or optionally substituted aryl residue,
R⁵ = H, NO₂, CN, OCH₃, halogen, alkyl or alkoxyalkyl having 1 to 4 C atoms or optionally substituted aryl residue,
X = SO₂ (sulfonyl), OCO (oxycarbonyl).

6. The method according to any one of the preceding claims, wherein the photolabile protecting group is NPPOC or CNPOC.

7. The method according to any one of the preceding claims, wherein the biochip is a nucleic acid chip or nucleic acid analogue chip.

8. The method according claim 7, wherein the nucleic acid chip is a DNA or RNA chip.

9. The method according to claim 7, wherein the nucleic acid analogue chip is a PNA or LNA chip.

10. The method according to any one of the preceding claims, wherein the condensation step common for the light-controlled chip synthesis is additionally carried out with pyridine hydrochloride as the catalyst.

## Revendications

1. Procédé de synthèse de biopuces commandée par la lumière avec utilisation de groupes protecteurs photolabiles du type 2-(2-nitrophényl)-éthyle, dans lequel l'étape d'irradiation classique dans la synthèse photolithographique de puces est conduite en présence d'une base.

2. Procédé suivant la revendication 1, dans lequel la base est une base secondaire ou tertiaire.

3. Procédé suivant la revendication 1 ou la revendication 2, dans lequel la base est choisie entre le DBU (1,8-diazabicyclo[5.4.0]undéc-7-ène, le DBN (1,5-diazabicycio[4.3.0]non-5-ène, la diisopropyléthylamine, la pyridine, la pipéridine, la triéthylamine, la diisopropylamine, la N-méthylmorpholine, la 2,6-lutidine, la collidine, le N-méthylimidazole, le DABCO, la N,N-diméthylaminopyridine.

4. Procédé suivant l'une des revendications 1 à 3, dans lequel la base est utilisée à une concentration de 0,01 à 1 M.

5. Procédé suivant l'une des revendications 1 à 4, dans lequel le groupe protecteur photolabile répond à la formule (I) : 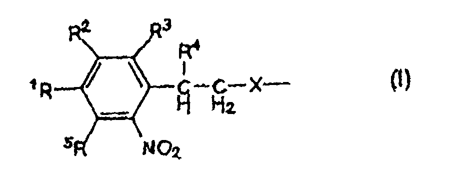 dans laquelle
R¹ représente H, NO₂, CN, OCH₃, un halogène, un reste alkyle ou alkoxyalkyle ayant 1 à 4 atomes de carbone,
R² représente H, OCH₃, un reste alkyle ayant 1 à 4 atomes de carbone ou un reste aryle éventuellement substitué,
R³ représente H, F, Cl, Br, NO₂, un reste alkyle ayant 1 à 4 atomes de carbone ou, le cas échéant, un reste aryle ou un reste acyle aliphatique ayant 2 à 5 atomes de carbone,
R⁴ représente H, un halogène, OCH₃, un reste alkyle ayant 1 à 4 atomes de carbone ou un reste aryle éventuellement substitué,
R⁵ représente H, NO₂, CN, OCH₃, un halogène, un reste alkyle ou alkoxyalkyle ayant 1 à 4 atomes de carbone ou un reste aryle éventuellement substitué,
X est un groupe SO₂ (sulfonyle), OCO (oxycarbonyle).

6. Procédé suivant l'une des revendications précédentes, dans lequel le groupe protecteur photolabile est un groupe NPPOC ou CNPOC.

7. Procédé suivant l'une des revendications précédentes, dans lequel la biopuce est une puce d'acide nucléique ou d'un analogue d'acide nucléique.

8. Procédé suivant la revendication 7, dans lequel la puce d'acide nucléique est une puce d'ADN ou d'ARN.

9. Procédé suivant la revendication 7, dans lequel la puce d'un analogue d'acide nucléique est une puce de PNA ou de LNA.

10. Procédé suivant l'une des revendications précédentes, dans lequel l'étape de condensation classique dans la synthèse de puces commandée par la lumière est conduite en outre avec le chlorhydrate de pyridine comme catalyseur.
